(19) Europäisches Patentamt
European Patent Office
Office européen des brevets

(11) Veröffentlichungsnummer: **0 040 345**
**B1**

(12) # EUROPÄISCHE PATENTSCHRIFT

(45) Veröffentlichungstag der Patentschrift: **11.07.84**

(21) Anmeldenummer: **81103322.4**

(22) Anmeldetag: **02.05.81**

(51) Int. Cl.³: **C 07 D 233/60**, C 07 D 249/08, C 07 D 301/02, C 07 D 303/02, C 07 D 303/22, A 01 N 43/50, A 01 N 43/64

(54) **1-Hydroxyethyl-azol-Derivate, Verfahren zu ihrer Herstellung sowie ihre Verwendung als Pflanzenwachstumsregulatoren und Fungizide.**

(30) Priorität: **16.05.80 DE 3018866**
**19.02.81 DE 3106076**

(43) Veröffentlichungstag der Anmeldung: **25.11.81 Patentblatt 81/47**

(45) Bekanntmachung des Hinweises auf die Patenterteilung: **11.07.84 Patentblatt 84/28**

(84) Benannte Vertragsstaaten: **AT BE CH DE FR GB IT LI NL SE**

(56) Entgegenhaltungen:
**AT - B - 346 850**
**CH - A - 614 201**
**DE - A - 2 654 890**
**DE - A - 2 736 122**
**US - A - 4 123 542**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Holmwood, Graham, Dr., Katernbergerstrasse 184, D-5600 Wuppertal 1 (DE)**
Erfinder: **Büchel, Karl Heinz, Prof. Dr., Dabringhausener Strasse 42, D-5093 Burscheid (DE)**
Erfinder: **Lürssen, Klaus, Dr., August-Kierspel-Strasse 89, D-5060 Bergisch-Gladbach 2 (DE)**
Erfinder: **Frohberger, Paul-Ernst, Dr., Willi-Baumeister-Strasse 5, D-5090 Leverkusen (DE)**
Erfinder: **Brandes, Wilhelm, Dr., Eichendorffstrasse 3, D-5653 Leichlingen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die Erfindung betrifft neue 1-Hydroxyethyl-azol-Derivate, ein Verfahren zu ihrer Herstellung sowie ihre Verwendung als Pflanzenwachstumsregulatoren und Fungizide.

Es sind bereits zahlreiche Triazol-Derivate mit pflanzenwuchsregulierenden und fungiziden Eigenschaften bekannt (vgl. DE-A-27 34 426 und DE-A-28 38 847). So lassen sich 1-(4-Chlorphenyl)-4,4-dimethyl-2-(1,2,4-triazol-1-yl)-pentan-3-on, 1-(4-Fluorphenyl)-4,4-dimethyl-2-(1,2,4-triazol-1-yl)-pent-1-en-3-ol und 1-(2,4-Dichlorphenyl)-4,4-dimethyl-2-(1,2,4-triazol-1-yl)-pent-1-en-3-ol als Pflanzenwachstumsregulatoren und Fungizide verwenden. Die Wirkung dieser Stoffe lässt allerdings bei niedrigen Aufwandmengen zu wünschen übrig.

Ausserdem ist bekannt, dass 2-Chlorethyl-trimethyl-ammoniumchlorid und 2-Chlorethyl-phosphonsäure pflanzenwuchsregulierende Eigenschaften besitzen (vgl. US-A-3 156 554 und DE-A-1 667 968). Die mit diesen Substanzen erzielten Ergebnisse sind aber ebenfalls nicht immer befriedigend.

Ferner ist bereits bekannt geworden, dass Zink-ethylen-1,2-bisdithiocarbamidat ein gutes Mittel zur Bekämpfung von pilzlichen Pflanzenkrankheiten ist [vergleiche Phytopathology *33,* 1113 (1963)]. Jedoch ist dessen Einsatz nur beschränkt möglich, da es insbesondere bei niedrigen Aufwandmengen und Konzentrationen nicht immer befriedigend wirksam ist.

Aus der DE-A-26 54 890 sind 1,2,4-Triazol-Derivate mit fungiziden Eigenschaften bekannt. Es werden jedoch keine Verbindungen beschrieben, in denen der Azol-Rest über eine Hydroxyethyl-Gruppe mit dem verbleibenden Molekülteil verbunden ist. In der DE-A-27 36 122 werden fungizid wirksame Imidazol-Derivate offenbart, in denen einer der beiden vorhandenen Phenyl-Reste über eine Methylen-Gruppe mit dem Kohlenstoffatom verknüpft ist, das eine Hydroxy-Gruppe trägt. Ausserdem werden in der US-A-4 123 542 Hydroxyethyl-imidazole erwähnt, die als Zwischenprodukte zur Synthese von Verbindungen mit fungizider Wirksamkeit dienen.

Schliesslich sind aus der AT-B-346 850, der CH-A-614 201 und der US-A-4 123 542 Oxirane bekannt. Stoffe dieses Typs, in denen der Dreiring an einem der beiden Kohlenstoffatome sowohl einen Phenethyl- bzw. Phenoxymethyl-Rest als auch einen der im folgenden als $R^1$ bezeichneten Reste enthält, werden jedoch nicht beschrieben.

Es wurden nun neue 1-Hydroxyethyl-azol-Derivate der allgemeinen Formel I

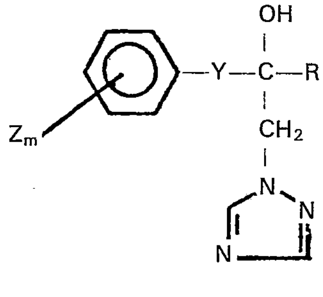

(I)

in welcher

R für Alkyl mit 1 bis 4 Kohlenstoffatomen, gegebenenfalls durch Alkyl mit 1 oder 2 Kohlenstoffatomen substituiertes Cycloalkyl mit 3 bis 7 Kohlenstoffatomen oder für gegebenenfalls durch Halogen, Alkyl mit 1 bis 4 Kohlenstoffatomen und/oder Halogenalkyl mit 1 oder 2 Kohlenstoffatomen und 1 bis 5 Halogenatomen substituiertes Phenyl steht,

Y für die Gruppierungen $-CH_2-CH_2-$ und $-O-CH_2-$ steht, wobei im Falle der $-OCH_2$-Gruppe das Sauerstoffatom mit dem Phenylrest verbunden ist,

Z für Halogen, Alkyl mit 1 bis 4 Kohlenstoffatomen, Cycloalkyl mit 5 bis 7 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, gegebenenfalls durch Halogen und/oder Alkyl mit 1 bis 4 Kohlenstoffatomen substituiertes Phenyl, gegebenenfalls durch Halogen und/oder Alkyl mit 1 bis 4 Kohlenstoffatomen substituiertes Phenoxy, gegebenenfalls durch Halogen und/oder Alkyl mit 1 bis 4 Kohlenstoffatomen substituiertes Phenylalkyl mit 1 oder 2 Kohlenstoffatomen im Alkylteil oder für gegebenenfalls durch Halogen und/oder Alkyl mit 1 bis 4 Kohlenstoffatomen substituiertes Phenylalkoxy mit 1 oder 2 Kohlenstoffatomen im Alkoxyteil steht und

m für die Zahlen 0, 1, 2 oder 3 steht,

sowie deren Säureadditions-Salze und Metallsalz-Komplexe gefunden.

Die Verbindungen der allgemeinen Formel I besitzen ein asymmetrisches Kohlenstoffatom und können deshalb in den beiden optischen Isomerenformen anfallen. Die Erfindung betrifft sowohl die Isomerengemische als auch die einzelnen Isomeren.

Weiterhin wurde gefunden, dass man die 1-Hydroxyethyl-azol-Derivate der allgemeinen Formel I erhält, wenn man Oxirane der allgemeinen Formel II

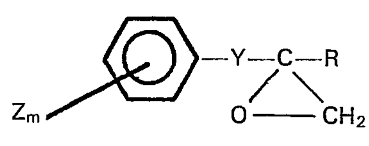

(II)

in welcher

R, Y, Z und m die oben angegebene Bedeutung haben, mit 1,2,4-Triazol der Formel III

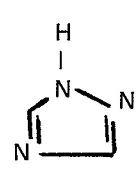

(III)

in Gegenwart eines Verdünnungsmittels und gegebenenfalls in Gegenwart einer Base umsetzt, und gegebenenfalls anschliessend an die erhaltenen Verbindungen der allgemeinen Formel I eine Säure oder ein Metallsalz addiert.

Ausserdem wurde gefunden, dass die neuen 1-Hydroxyethyl-azol-Derivate der allgemeinen Formel

I starke pflanzenwachstumsregulierende und starke fungizide Eigenschaften besitzen.

Überraschenderweise zeigen die erfindungsgemässen 1-Hydroxyethyl-azol-Derivate der allgemeinen Formel I eine bessere pflanzenwachstumsregulierende Wirkung als das bekannte 2-Chlorethyltrimethylammoniumchlorid und als die ebenfalls bekannte 2-Chlorethylphosphonsäure, welches anerkannt gut wirksame Stoffe gleicher Wirkungsart sind.

Ausserdem besitzen die erfindungsgemässen Verbindungen überraschenderweise eine bessere fungizide Wirkung, als das aus dem Stand der Technik bekannte Zink-ethylen-1,2-bisdithiocarbamidat, welches eine wirkungsmässig naheliegende Verbindung ist.

Die erfindungsgemässen 1-Hydroxyethyl-azol-Derivate der allgemeinen Formel I übertreffen überraschenderweise auch die konstitutionell ähnlichen vorbekannten Verbindungen 1-(4-Fluorphenyl)-4,4-dimethyl-2-(1,2,4-triazol-1-yl)-pent-1-en-3-ol und 1-(2,4-Dichlorphenyl)-4,4-dimethyl-2-(1,2,4-triazol-1-yl)-pent-1-en-3-ol bezüglich ihrer pflanzenwuchsregulierenden Eigenschaften. Darüber hinaus besitzen sie auch eine bessere fungizide Wirksamkeit als das 1-(4-Chlorphenyl)-4,4-dimethyl-2-(1,2,4-triazol-1-yl)-pentan-3-on, welches ebenfalls ein konstitutionell ähnlicher Stoff analoger Wirkungsrichtung ist.

Die erfindungsgemässen 1-Hydroxyethyl-azol-Derivate sind durch die allgemeine Formel I definiert.

Besonders bevorzugt sind diejenigen Verbindungen der allgemeinen Formel I, in denen R für tert.-Butyl, Isopropyl oder Methyl steht, für jeweils gegebenenfalls durch Methyl substituiertes Cyclopropyl, Cyclopentyl oder Cyclohexyl steht sowie für gegebenenfalls einfach oder zweifach gleich oder verschieden durch Fluor, Chlor, Methyl oder Trifluormethyl substituiertes Phenyl steht; Z für Fluor, Chlor, Brom, Methyl, tert.-Butyl, Cyclohexyl, Methoxy, Methylthio, Trifluormethyl, Trifluormethoxy, Trifluormethylthio, sowie für jeweils gegebenenfalls einfach oder zweifach, gleich oder verschieden durch Fluor, Chlor und/oder Methyl substituiertes Phenyl, Phenoxy, Benzyl oder Benzyloxy steht, und Y sowie der Index m die in der Erfindungsdefinition angegebene Bedeutung haben.

Im einzelnen seien ausser den bei den Herstellungsbeispielen genannten Verbindungen folgende Verbindungen der allgemeinen Formel I genannt:

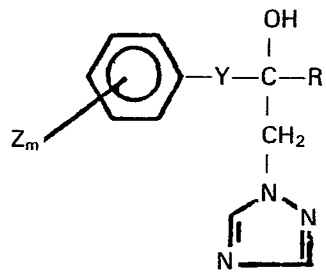

(I)

*Tabelle 1*

| $Z_m$ | Y | R |
|---|---|---|
| 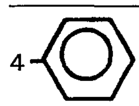 | $-O-CH_2-$ | $-C(CH_3)_3$ |
| 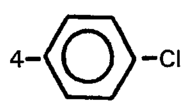 | » | » |
| 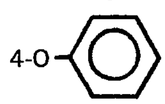 | » | » |
| 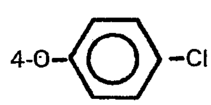 | » | » |
| 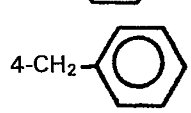 | » | » |
| 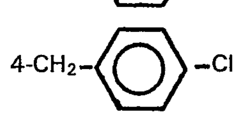 | » | » |
| 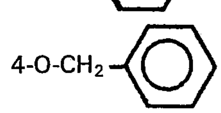 | » | » |
| 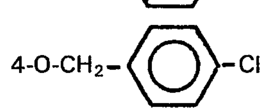 | » | » |

*Tabelle 1 (Fortsetzung)*

| $Z_m$ | Y | R |
|---|---|---|
| 3,4-$Cl_2$ | -O-$CH_2$- | -$C(CH_3)_3$ |
| 4-$CF_3$ | » | » |
| 4-$OCF_3$ | » | » |
| 4-$SCF_3$ | » | » |
| 4-$SCH_3$ | » | » |
| 4-$C(CH_3)_3$ | » | » |
| 4-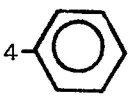 | O-$CH_2$ | 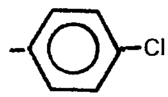 |
| 4-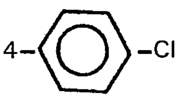 | » | » |
| 4-O-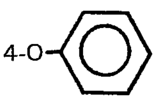 | » | » |
| 4-O-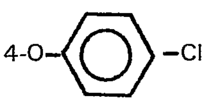 | » | » |
| 4-$CH_2$-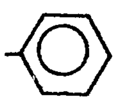 | » | » |
| 4-$CH_2$-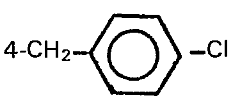 | » | » |
| 4-O-$CH_2$-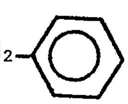 | » | » |
| 4-O-$CH_2$-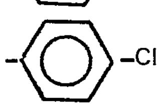 | » | » |
| 3,4-$Cl_2$ | » | » |
| 4-$CF_3$ | » | » |
| 4-$OCF_3$ | » | » |

*Tabelle 1 (Fortsetzung)*

| $Z_m$ | Y | R |
|---|---|---|
| $4-SCF_3$ | $O-CH_2$ | 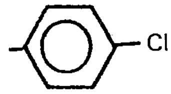 |
| $4-SCH_3$ | » | » |
| $4-C(CH_3)_3$ | » | » |
| 4 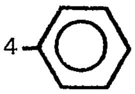 | $-O-CH_2-$ | $-CH(CH_3)_2$ |
| 4 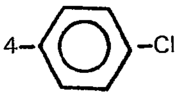 | » | » |
| 4-O 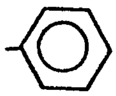 | » | » |
| 4-O 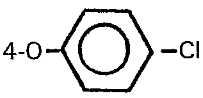 | » | » |
| $4-CH_2$ 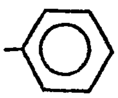 | » | » |
| $4-CH_2$ 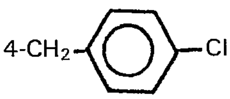 | » | » |
| $4-O-CH_2$ 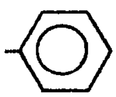 | » | » |
| $4-O-CH_2$ 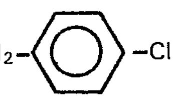 | » | » |
| $3,4-Cl_2$ | » | » |
| $4-CF_3$ | » | » |
| $4-OCF_3$ | » | » |
| $4-SCF_3$ | » | » |
| $4-SCH_3$ | » | » |
| $4-C(CH_3)_3$ | » | » |

*Tabelle 1 (Fortsetzung)*

| $Z_m$ | Y | R |
|---|---|---|
| 4- 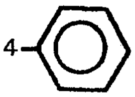 | -O-$CH_2$- | 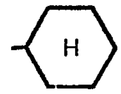 |
| 4- 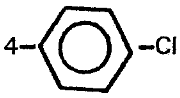 | » | » |
| 4-O 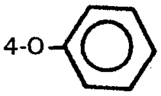 | » | » |
| 4-O 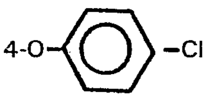 | » | » |
| 4-$CH_2$ 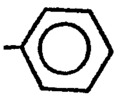 | » | » |
| 4-$CH_2$ 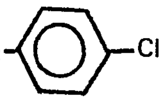 | » | » |
| 4-O-$CH_2$ 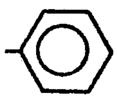 | » | » |
| 4-O-$CH_2$ 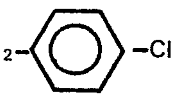 | » | » |
| 3,4-$Cl_2$ | » | » |
| 4-$CF_3$ | » | » |
| 4-$OCF_3$ | » | » |
| 4-$SCF_3$ | » | » |
| 4-$SCH_3$ | » | » |
| 4-$C(CH_3)_3$ | » | » |
| 4- 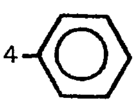 | -O-$CH_2$- | 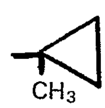 |
| 4- 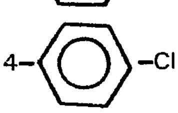 | » | » |
| 4-O 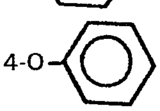 | » | » |

*Tabelle 1 (Fortsetzung)*

| $Z_m$ | Y | R |
|---|---|---|
| 4-O-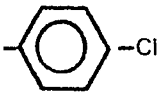 | -O-$CH_2$- | 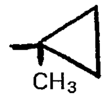 |
| 4-$CH_2$-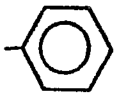 | » | » |
| 4-$CH_2$-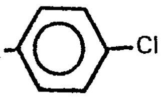 | » | » |
| 4-O-$CH_2$-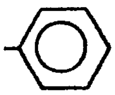 | » | » |
| 4-O-$CH_2$-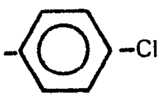 | » | » |
| 3,4-$Cl_2$ | » | » |
| 4-$CF_3$ | » | » |
| 4-$OCF_3$ | » | » |
| 4-$SCF_3$ | » | » |
| 4-$SCH_3$ | » | » |
| 4-$C(CH_3)_3$ | » | » |
| 4-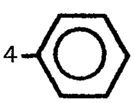 | -$CH_2$-$CH_2$- | -$C(CH_3)_3$ |
| 4-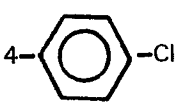 | » | » |
| 4-O-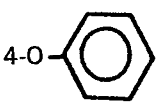 | » | » |
| 4-O-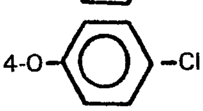 | » | » |
| 4-$CH_2$-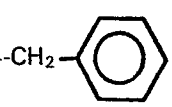 | » | » |
| 4-$CH_2$-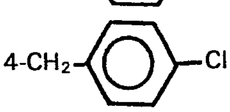 | » | » |

*Tabelle 1 (Fortsetzung)*

| $Z_m$ | Y | R |
|---|---|---|
| 4-O-$CH_2$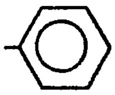 | $-CH_2-CH_2-$ | $-C(CH_3)_3$ |
| 4-O-$CH_2$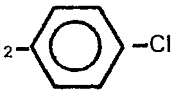 | » | » |
| 3,4-$Cl_2$ | » | » |
| 4-$CF_3$ | » | » |
| 4-$OCF_3$ | » | » |
| 4-$SCF_3$ | » | » |
| 4-$SCH_3$ | » | » |
| 4-$C(CH_3)_3$ | » | » |
| 4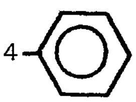 | $-CH_2-CH_2-$ | 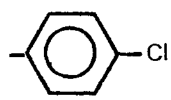 |
| 4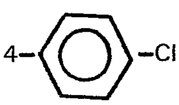 | » | » |
| 4-O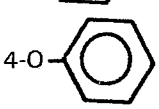 | » | » |
| 4-O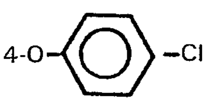 | » | » |
| 4-$CH_2$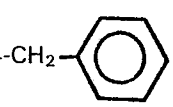 | » | » |
| 4-$CH_2$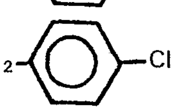 | » | » |
| 4-O-$CH_2$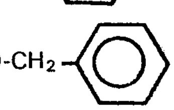 | » | » |
| 4-O-$CH_2$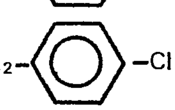 | » | » |
| 3,4-$Cl_2$ | » | » |

*Tabelle 1 (Fortsetzung)*

| $Z_m$ | Y | R |
|---|---|---|
| $4\text{-}CF_3$ | $-CH_2\text{-}CH_2-$ | 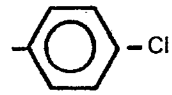 |
| $4\text{-}OCF_3$ | » | » |
| $4\text{-}SCF_3$ | » | » |
| $4\text{-}SCH_3$ | » | » |
| $4\text{-}C(CH_3)_3$ | » | » |
| 4- 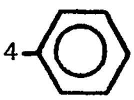 | $-CH_2\text{-}CH_2-$ | $-CH(CH_3)_2$ |
| 4- 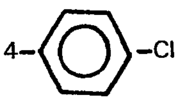 | » | » |
| 4-O- 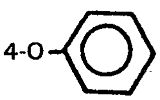 | » | » |
| 4-O- 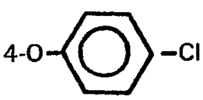 | » | » |
| $4\text{-}CH_2-$ 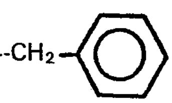 | » | » |
| $4\text{-}CH_2-$ 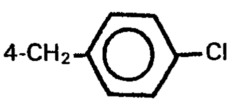 | » | » |
| $4\text{-}O\text{-}CH_2-$ 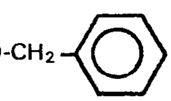 | » | » |
| $4\text{-}O\text{-}CH_2-$ 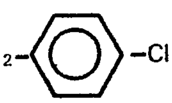 | » | » |
| $3,4\text{-}Cl_2$ | » | » |
| $4\text{-}CF_3$ | » | » |
| $4\text{-}OCF_3$ | » | » |
| $4\text{-}SCF_3$ | » | » |

*Tabelle 1 (Fortsetzung)*

| $Z_m$ | Y | R |
|---|---|---|
| 4-$SCH_3$ | $-CH_2-CH_2-$ | $-CH(CH_3)_2$ |
| 4-$C(CH_3)_3$ | » | » |
| 4-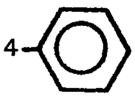 | $-CH_2-CH_2-$ | 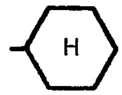 |
| 4-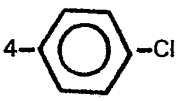 | » | » |
| 4-O-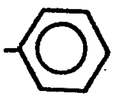 | » | » |
| 4-O-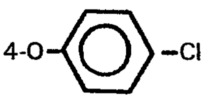 | » | » |
| 4-$CH_2$-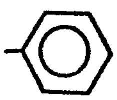 | » | » |
| 4-$CH_2$-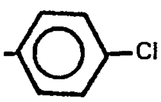 | » | » |
| 4-O-$CH_2$-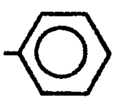 | » | » |
| 4-O-$CH_2$-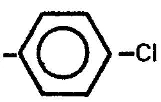 | » | » |
| 3,4-$Cl_2$ | » | » |
| 4-$CF_3$ | » | » |
| 4-$OCF_3$ | » | » |
| 4-$SCF_3$ | » | » |
| 4-$SCH_3$ | » | » |
| 4-$C(CH_3)_3$ | » | » |
| 4-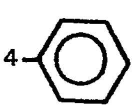 | $-CH_2-CH_2-$ | 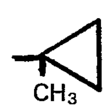 |

*Tabelle 1 (Fortsetzung)*

| $Z_m$ | Y | R |
|---|---|---|
| 4-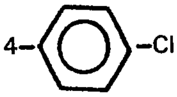Cl | $-CH_2-CH_2-$ | 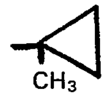 |
| 4-O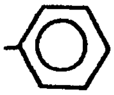 | » | » |
| 4-O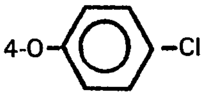Cl | » | » |
| $4-CH_2$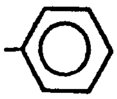 | » | » |
| $4-CH_2$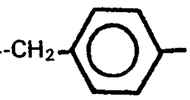Cl | » | » |
| $4-O-CH_2$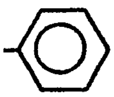 | » | » |
| $4-O-CH_2$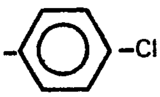Cl | » | » |
| $3,4-Cl_2$ | » | » |
| $4-CF_3$ | » | » |
| $4-OCF_3$ | » | » |
| $4-SCF_3$ | » | » |
| $4-SCH_3$ | » | » |
| $4-C(CH_3)_3$ | » | » |
| 4-Cl | $-O-CH_2-$ | $-CH(CH_3)_2$ |
| 4-F | » | » |
| $4-CH_3$ | » | » |
| 4-Cl | $-O-CH_2-$ | 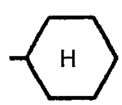 |

*Tabelle 1 (Fortsetzung)*

| $Z_m$ | Y | R |
|---|---|---|
| 4-F | $-O-CH_2-$ | 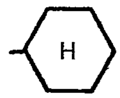 |
| $4-CH_3$ | » | » |
| 4-Cl | $-O-CH_2-$ | 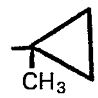 |
| 4-F | » | » |
| $4-CH_3$ | » | » |
| 4-Cl | $-CH_2-CH_2-$ | $-CH(CH_3)_2$ |
| 4-F | » | » |
| $4-CH_3$ | » | » |
| 4-Cl | $-CH_2-CH_2-$ | 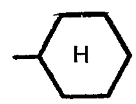 |
| 4-F | » | » |
| $4-CH_3$ | » | » |
| 4-Cl | $-CH_2-CH_2$ | 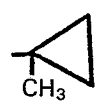 |
| 4-F | » | » |
| $4-CH_3$ | » | » |
| $2,4-Cl_2$ | $-CH_2-CH_2-$ | $-C(CH_3)_3$ |
| $4-CH_3$ | » | » |
| $4-Cl, 2-CH_3$ | » | » |

*Tabelle 1 (Fortsetzung)*

| $Z_m$ | Y | R |
|---|---|---|
| 4-F | $-O-CH_2-$ | $-C(CH_3)_3$ |
| $2-CH_3$ | $-CH_2-CH_2-$ | $-C(CH_3)_3$ |

Verwendet man beispielsweise 2-(4-Chlorphenoxy-methyl)-2-tert.-butyl-oxiran und 1,2,4-Triazol als Ausgangsstoffe, so kann der Ablauf des erfindungsgemässen Verfahrens durch das folgende Formelschema wiedergegeben werden:

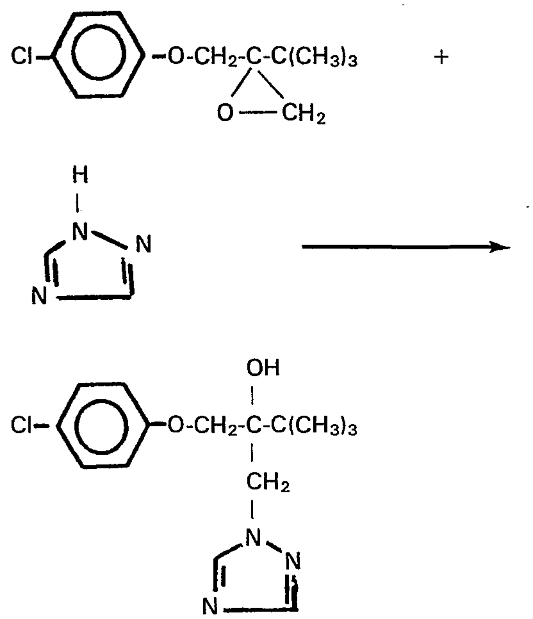

Die bei der Durchführung des erfindungsgemässen Verfahrens als Ausgangsstoffe zu verwendenden Oxirane sind durch die allgemeine Formel II definiert. In dieser Formel haben R, Y, Z und der Index m die Bedeutungen, die bereits im Zusammenhang mit der Beschreibung der erfindungsgemässen Stoffe der allgemeinen Formel I für diese Substituenten bzw. für den Index m genannt wurden.

Die Oxirane der allgemeinen Formel IIa

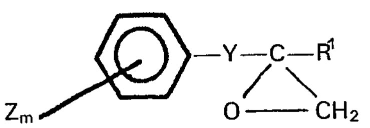

(IIa)

in welcher

$R^1$ für tert.-Butyl, gegebenenfalls durch Alkyl mit 1 oder 2 Kohlenstoffatomen substituiertes Cycloalkyl mit 3 bis 7 Kohlenstoffatomen oder für gegebenenfalls durch Halogen, Alkyl mit 1 bis 4 Kohlenstoffatomen und/oder Halogenalkyl mit 1 oder 2 Kohlenstoffatomen und 1 bis 5 Halogenatomen substituiertes Phenyl steht,

Y für die Gruppierungen $-CH_2-CH_2$ und $-O-CH_2$ steht, wobei im Falle der $-OCH_2$-Gruppe das Sauerstoffatom mit dem Phenylrest verbunden ist,

Z für Halogen, Alkyl mit 1 bis 4 Kohlenstoffatomen, Cycloalkyl mit 5 bis 7 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, gegebenenfalls durch Halogen und/oder Alkyl mit 1 bis 4 Kohlenstoffatomen substituiertes Phenyl, gegebenenfalls durch Halogen und/oder Alkyl mit 1 bis 4 Kohlenstoffatomen substituiertes Phenoxy, gegebenenfalls durch Halogen und/oder Alkyl mit 1 bis 4 Kohlenstoffatomen substituiertes Phenylalkyl mit 1 oder 2 Kohlenstoffatomen im Alkylteil oder für gegebenenfalls durch Halogen und/oder Alkyl mit 1 bis 4 Kohlenstoffatomen substituiertes Phenylalkoxy mit 1 oder 2 Kohlenstoffatomen im Alkoxyteil steht und

m für die Zahlen 0, 1, 2 oder 3 steht,

sind neu.

Die Oxirane der allgemeinen Formel IIa lassen sich herstellen, indem man Ketone der allgemeinen Formel IV

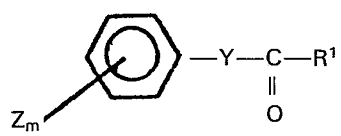

(IV)

in welcher

$R^1$, Y, Z und m die oben angegebene Bedeutung haben, entweder

α) mit Dimethyloxosulfonium-methylid der Formel V

$$\overset{\delta+\ \delta-}{(CH_3)_2SOCH_2} \qquad (V)$$

in Gegenwart eines Verdünnungsmittels umsetzt, oder

β) mit Trimethylsulfonium-methylsulfat der Formel VI

$$\left[\overset{(+)}{(CH_3)_3S}\right] \quad \overset{(-)}{CH_3SO_4} \qquad (VI)$$

in Gegenwart eines inerten organischen Lösungsmittels sowie in Gegenwart einer Base umsetzt.

Die bei der Herstellung der Oxirane der allgemeinen Formel IIa als Ausgangsstoffe benötigten Ketone der allgemeinen Formel IV sind bekannt [vgl. DE-PS 22 01 063, DE-OS 27 05 678, DE-OS 27 37 489, Tetrahedron *31,* 3 (1975) und Chemical Abstracts *84,* 73 906 n] oder lassen sich nach im Prinzip bekannten Verfahren herstellen.

Das bei der Verfahrensvariante ($\alpha$) benötigte Dimethyloxosulfonium-methylid der Formel V ist ebenfalls bekannt [vgl. J. Amer. Chem. Soc. *87,* 1363--1364 (1965)]. Es wird bei der obigen Umsetzung in frisch zubereitetem Zustand verarbeitet, indem man es in situ durch Umsetzung von Trimethyloxosulfoniumjodid mit Natriumhydrid bzw. Natriumamid in Gegenwart eines Verdünnungsmittels erzeugt.

Das bei der Verfahrensvariante ($\beta$) benötigte Trimethylsulfonium-methylsulfat der Formel VI ist ebenfalls bekannt [vgl. Heterocycles *8,* 397 (1977)]. Es wird bei der obigen Umsetzung ebenfalls in frisch hergestelltem Zustand eingesetzt, indem man es durch Reaktion von Dimethylsulfid mit Dimethylsulfat in situ erzeugt.

Bei der Variante ($\alpha$) des Verfahrens zur Herstellung der Oxirane der allgemeinen Formel IIa kommt als Verdünnungsmittel vorzugsweise Dimethylsulfoxid in Frage.

Die Reaktionstemperaturen können bei der oben beschriebenen Verfahrensvariante ($\alpha$) innerhalb eines grösseren Bereiches variiert werden. Im allgemeinen arbeitet man bei Temperaturen zwischen 20 und 80°C.

Die Durchführung des Verfahrens zur Herstellung der Oxirane der allgemeinen Formel IIa nach der Variante ($\alpha$) und die Aufarbeitung des bei dieser Synthese anfallenden Reaktionsgemisches erfolgen nach üblichen Methoden [vgl. J. Amer. Chem. Soc. *87,* 1363-1364 (1965)].

Bei der Variante ($\beta$) zur Herstellung der Oxirane der allgemeinen Formel IIa kommt als inertes organisches Lösungsmittel vorzugsweise Acetonitril in Betracht.

Als Basen können bei der Verfahrensvariante ($\beta$) starke anorganische oder organische Basen verwendet werden. Vorzugsweise in Frage kommt Natriummethylat.

Die Reaktionstemperaturen können bei der oben beschriebenen Verfahrensvariante ($\beta$) innerhalb eines bestimmten Bereiches variiert werden. Im allgemeinen arbeitet man bei Temperaturen zwischen 0 und 60°C, vorzugsweise bei Raumtemperatur.

Die Durchführung des Verfahrens zur Herstellung der Oxirane der allgemeinen Formel IIa nach der Variante ($\beta$) und die Aufarbeitung des bei dieser Synthese anfallenden Reaktionsproduktes erfolgen nach üblichen Methoden [vgl. Heterocycles *8,* 397 (1977)].

Die bei dem erfindungsgemässen Verfahren als Ausgangsstoffe benötigten Oxirane, die nicht von der allgemeinen Formel IIa umfasst werden, lassen sich ebenfalls nach den oben beschriebenen Verfahrensvarianten ($\alpha$) und ($\beta$) aus den entsprechenden Ketonen herstellen.

Die Oxirane der allgemeinen Formel II können bei dem erfindungsgemässen Verfahren gegebenenfalls ohne Isolierung direkt weiter umgesetzt werden.

Als Verdünnungsmittel kommen für die erfindungsgemässe Umsetzung alle inerten organischen Lösungsmittel in Frage. Hierzu gehören vorzugsweise Alkohole, wie z.B. Ethanol und Methoxyethanol; Ketone, wie z.B. 2-Butanon; Nitrile, wie z.B. Acetonitril; Ester, wie z.B. Essigester; Ether, wie z.B. Dioxan; aromatische Kohlenwasserstoffe, wie z.B. Benzol und Toluol; oder Amide, wie z.B. Dimethylformamid.

Als Basen kommen für die erfindungsgemässe Umsetzung alle üblicherweise verwendbaren organischen und anorganischen Basen in Betracht. Hierzu gehören vorzugsweise Alkalicarbonate, wie z.B. Natrium- oder Kaliumcarbonat; Alkalihydroxide, wie z.B. Natriumhydroxid; Alkalialkoholate, wie z.B. Natrium- und Kaliummethylat und -ethylat; Alkalihydride, wie z.B. Natriumhydrid; sowie niedere tertiäre Alkylamine, Cycloalkylamine und Aralkylamine, wie insbesondere Triethylamin.

Die Reaktionstemperaturen können bei der Durchführung des erfindungsgemässen Verfahrens in einem grösseren Bereich variiert werden. Im allgemeinen arbeitet man bei Temperaturen zwischen 0 und 200°C, vorzugsweise zwischen 60 und 150°C.

Die erfindungsgemässe Umsetzung kann gegebenenfalls unter erhöhtem Druck durchgeführt werden. Im allgemeinen arbeitet man zwischen 1 und 50 bar, vorzugsweise zwischen 1 und 25 bar.

Bei der Durchführung des erfindungsgemässen Verfahrens setzt man auf 1 Mol Oxiran der allgemeinen Formel II vorzugsweise 1 bis 2 Mol 1,2,4--Triazol und gegebenenfalls 1 bis 2 Mol Base ein. Die Isolierung der Endprodukte erfolgt in allgemein üblicher Weise.

Die nach dem erfindungsgemässen Verfahren erhältlichen Verbindungen der allgemeinen Formel I können in Säureadditions-Salze bzw. Metallsalz--Komplexe überführt werden.

Zur Herstellung von physiologisch verträglichen Säureadditionssalzen der Verbindungen der allgemeinen Formel I kommen vorzugsweise folgende Säuren in Frage: Die Halogenwasserstoffsäuren, wie z.B. Chlorwasserstoffsäure und Bromwasserstoffsäure, insbesondere Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Citronensäure, Salicylsäure, Sorbinsäure, Milchsäure sowie Sulfonsäuren, wie z.B. p-Toluolsulfonsäure und 1,5-Naphthalindisulfonsäure.

Die Säureadditions-Salze der Verbindungen der allgemeinen Formel I können in einfacher Weise nach üblichen Salzbildungsmethoden, z.B. durch Lösung einer Verbindung der allgemeinen Formel I in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der allgemeinen Formel I kommen vorzugsweise Salze von Metallen der II. bis IV. Hauptgruppe und der I. und II. sowie IV. bis VIII. Nebengruppe des Periodensystems in Frage, wobei Kupfer, Zink, Mangan, Magnesium, Zinn, Eisen und Nickel beispielhaft genannt seien.

Als Anionen der Salze kommen solche in Betracht, die sich vorzugsweise von folgenden Säuren ableiten: Halogenwasserstoffsäuren, wie z.B. Chlorwasserstoffsäure und Bromwasserstoffsäure, ferner Phosphorsäure, Salpetersäure und Schwefelsäure.

Die Metallsalz-Komplexe von Verbindungen der allgemeinen Formel I können in einfacher Weise nach üblichen Verfahren erhalten werden, so z.B. durch Lösen des Metallsalzes in Alkohol, z.B. Ethanol und Hinzufügen zur Verbindung der allgemeinen Formel I. Man kann Metallsalz-Komplexe in bekannter Weise, z.B. durch Abfiltrieren isolieren und gegebenenfalls durch Umkristallisieren reinigen.

Die erfindungsgemässen Wirkstoffe greifen in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, dass ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Pflanzenwuchsregulierende Stoffe können zum Beispiel zur Hemmung des vegetativen Wachstums der Pflanzen eingesetzt werden. Eine derartige Wuchshemmung ist unter anderem bei Gräsern von wirtschaftlichem Interesse, denn dadurch kann die Häufigkeit der Grasschnitte in Ziergärten, Park- und Sportanlagen, an Strassenrändern, auf Flughäfen oder in Obstanlagen reduziert werden. Von Bedeutung ist auch die Hemmung des Wuchses von krautigen und holzigen Pflanzen an Strassenrändern und in der Nähe von Pipelines oder Überlandleitungen oder ganz allgemein in Bereichen, in denen ein starker Zuwachs der Pflanzen unerwünscht ist.

Wichtig ist auch die Anwendung von Wachstumsregulatoren zur Hemmung des Längenwachstums von Getreide. Hierdurch wird die Gefahr des Umknickens («Lagerns») der Pflanzen vor der Ernte verringert oder vollkommen beseitigt. Ausserdem können Wachstumsregulatoren bei Getreide eine Halmverstärkung hervorrufen, die ebenfalls dem Lagern entgegenwirkt. Die Anwendung von Wachstumsregulatoren zur Halmverkürzung und Halmverstärkung erlaubt es, höhere Düngermengen auszubringen, um den Ertrag zu steigern, ohne dass die Gefahr besteht, dass das Getreide lagert.

Eine Hemmung des vegetativen Wachstums ermöglicht bei vielen Kulturpflanzen eine dichtere Anpflanzung, so dass Mehrerträger bezogen auf die Bodenfläche erzielt werden können. Ein Vorteil der so erzielten kleineren Pflanzen ist auch, dass die Kultur leichter bearbeitet und geerntet werden kann.

Eine Hemmung des vegetativen Wachstums der Pflanzen kann auch dadurch zu Ertragssteigerungen führen, dass die Nährstoffe und Assimilate in stärkerem Masse der Blüten- und Fruchtbildung zugute kommen als den vegetativen Pflanzenteilen.

Mit Wachstumsregulatoren lässt sich häufig auch eine Förderung des vegetativen Wachstums erzielen. Dies ist von grossem Nutzen, wenn die vegetativen Pflanzenteile geerntet werden. Eine Förderung des vegetativen Wachstums kann aber auch gleichzeitig zu einer Förderung des generativen Wachstums führen, dadurch dass mehr Assimilate gebildet werden, so dass mehr oder grössere Früchte entstehen.

Ertragssteigerungen können in manchen Fällen durch einen Eingriff in den pflanzlichen Stoffwechsel erreicht werden, ohne dass sich Änderungen des vegetativen Wachstums bemerkbar machen. Ferner kann mit Wachstumsregulatoren eine Veränderung der Zusammensetzung der Pflanzen erreicht werden, was wiederum zu einer Qualitätsverbesserung der Ernteprodukte führen kann. So ist es beispielsweise möglich, den Gehalt an Zucker in Zuckerrüben, Zuckerrohr, Ananas sowie in Zitrusfrüchten zu erhöhen oder den Proteingehalt in Soja oder Getreide zu steigern. Auch ist es beispielsweise möglich, den Abbau erwünschter Inhaltsstoffe, wie z.B. Zucker in Zuckerrüben oder Zuckerrohr, mit Wachstumsregulatoren vor oder nach der Ernte zu hemmen. Ausserdem lässt sich die Produktion oder der Abfluss von sekundären Pflanzeninhaltsstoffen positiv beeinflussen. Als Beispiel sei die Stimulierung des Latexflusses bei Gummibäumen genannt.

Unter dem Einfluss von Wachstumsregulatoren kann es zur Ausbildung parthenokarper Früchte kommen. Ferner kann das Geschlecht der Blüten beeinflusst werden. Auch kann eine Sterilität des Pollens erzeugt werden, was bei der Züchtung und Herstellung von Hybridsaatgut eine grosse Bedeutung hat.

Durch den Einsatz von Wachstumsregulatoren lässt sich die Verzweigung der Pflanzen steuern. Einerseits kann durch Brechen der Apikaldominanz die Entwicklung von Seitentrieben gefördert werden, was besonders im Zierpflanzenbau auch in Verbindung mit einer Wuchshemmung sehr erwünscht sein kann. Andererseits ist es aber auch möglich, das Wachstum der Seitentriebe zu hemmen. Für diese Wirkung besteht z.B. grosses Interesse im Tabakanbau oder bei der Anpflanzung von Tomaten.

Unter dem Einfluss von Wachstumsregulatoren kann der Blattbestand der Pflanzen so gesteuert werden, dass ein Entblättern der Pflanzen zu einem gewünschten Zeitpunkt erreicht wird. Eine derartige Entlaubung spielt bei der mechanischen Beerntung der Baumwolle eine grosse Rolle ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Ebenso lässt sich mit Wachstumsregulatoren der Fruchtfall steuern. Einerseits kann ein vorzeitiger Fruchtfall verhindert werden. Andererseits kann aber auch der Fruchtfall oder sogar das Abfallen der Blüten bis zu einem gewünschten Masse gefördert werden («Ausdünnung»), um die Alternanz zu brechen. Unter Alternanz versteht man die Eigenart einiger Obstarten, endogen bedingt von Jahr zu Jahr sehr unterschiedliche Erträge zu bringen. Schliesslich ist es möglich, mit Wachstumsregulatoren zum Zeitpunkt der Ernte die zum Ablösen der Früchte erforderlichen Kräfte zu reduzieren, um eine mechanische Beerntung zu ermöglichen oder eine manuelle Beerntung zu erleichtern.

Mit Wachstumsregulatoren lässt sich ferner eine Beschleunigung oder auch Verzögerung der Reife des Erntegutes vor oder nach der Ernte erreichen. Dieses ist von besonderem Vorteil, weil sich dadurch eine optimale Anpassung an die Bedürfnisse des Marktes herbeiführen lässt. Weiterhin können Wachstumsregulatoren in manchen Fällen die Fruchtausfärbung verbessern. Darüber hinaus kann mit Wachstumsregulatoren auch eine zeitliche Konzentrierung der Reife erzielt werden. Damit werden die Voraussetzungen dafür geschaffen, dass z.B. bei Tabak, Tomaten oder Kaffee eine vollständige mechanische oder manuelle Beerntung in einem Arbeitsgang vorgenommen werden kann.

Durch Anwendung von Wachstumsregulatoren kann ferner die Samen- oder Knospenruhe der Pflanzen beeinflusst werden, so dass die Pflanzen, wie z.B. Ananas oder Zierpflanzen in Gärtnereien, zu einem Zeitpunkt keimen, austreiben oder blühen, an dem sie normalerweise hierzu keine Bereitschaft zeigen. Eine Verzögerung des Austriebes von Knospen oder der Keimung von Samen mit Hilfe von Wachstumsregulatoren kann in frostgefährdeten Gebieten erwünscht sein, um Schädigungen durch Spätfröste zu vermeiden.

Schliesslich kann mit Wachstumsregulatoren eine Resistenz der Pflanzen gegen Frost, Trockenheit oder hohen Salzgehalt des Bodens induziert werden. Hierdurch wird die Kultivierung von Pflanzen in Gebieten möglich, die hierzu normalerweise ungeeignet sind.

Die erfindungsgemässen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäss verwendbaren Wirkstoffe mit besonders gutem Erfolg zur Bekämpfung solcher Pilze eingesetzt werden, die echte Mehltauerkrankungen hervorrufen; so zur Bekämpfung von Erysiphe-Arten, wie z.B. gegen den Erreger des Gersten- bzw. des Getreidemehltaus (Erysiphe graminis).

Besonders hervorzuheben ist, dass die erfindungsgemässen Wirkstoffe nicht nur eine protektive Wirkung entfalten, sondern auch systemisch wirksam sind. So gelingt es, Pflanzen gegen Pilzbefall zu schützen, wenn man den Wirkstoff über den Boden und die Wurzel oder über das Saatgut den oberirdischen Teilen der Pflanze zuführt.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengel. Als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweisshydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie

Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemässen Wirkstoffe können in den Formulierungen in Mischung mit anderen bekannten Wirkstoffen vorliegen, wie Fungizide, Insektizide, Akarizide und Herbizide, sowie in Mischungen mit Düngermitteln und anderen Wachstumsregulatoren.

Die Wirkstoffe können als solche, in Form ihrer Formulierungen oder der daraus bereiteten Anwendungsformen, wie gebrauchsfertige Lösungen, emulgierbare Konzentrate, Emulsionen, Schäume, Suspensionen, Spitzpulver, Pasten, lösliche Pulver, Stäubemittel und Granulate, angewendet werden. Die Anwendung geschieht in üblicher Weise, z.B. durch Giessen, Verspritzen, Versprühen, Verstreuen, Verstäuben, Verschäumen, Bestreichen usw.

Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemässen Verbindungen als Pflanzenwachstumsregulatoren können die Aufwandmengen in einem grösseren Bereich variiert werden. Im allgemeinen verwendet man pro Hektar Bodenfläche 0,01 bis 50 kg, bevorzugt 0,05 bis 10 kg an Wirkstoff.

Beim Einsatz der erfindungsgemässen Stoffe als Pflanzenwachstumsregulatoren gilt, dass die Anwendung in einem bevorzugten Zeitraum vorgenommen wird, dessen genaue Abgrenzung sich nach den klimatischen und vegetativen Gegebenenheiten richtet.

Auch beim Einsatz der erfindungsgemässen Stoffe als Fungizide kann die Aufwandmenge je nach Art der Applikation in einem grösseren Bereich variiert werden. So liegen die Wirkstoffkonzentrationen bei der Behandlung von Pflanzenteilen in den Anwendungsformen im allgemeinen zwischen 1 und 0,0001 Gew.-%, vorzugsweise zwischen 0,5 und 0,001%. Bei der Saatgutbehandlung werden im allgemeinen Wirkstoffmengen von 0,001 bis 50 g je Kilogramm Saatgut, vorzugsweise 0,01 bis 10 g, benötigt. Bei Behandlung des Bodens sind Wirkstoffkonzentrationen von 0,00001 bis 0,1 Gew.-%, vorzugsweise von 0,0001 bis 0,02%, am Wirkungsort erforderlich.

*Herstellungsbeispiele*

*Beispiel 1*

(I-1)

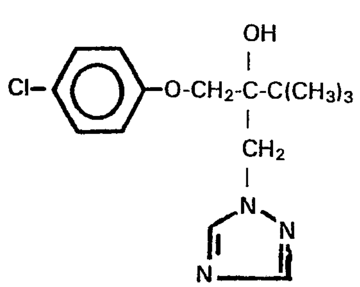

72,15 g (0,3 Mol) 2-(4-Chlorphenoxy-methyl)-2-tert.-butyl-oxiran und 24,15 g (0,35 Mol) 1,2,4-Triazol werden in 120 ml Ethanol 48 Stunden unter Rückfluss erhitzt. Anschliessend wird eingeengt, der Rückstand in 200 ml Essigester aufgenommen und erhitzt. Danach wird im Eisbad abgekühlt, der Feststoff abgesaugt und mit Essigester nachgewaschen. Das Filtrat wird eingeengt, der Rückstand in Ether/-Hexan gelöst und mit Chlorwasserstoff begast. Man saugt den Niederschlag ab, wäscht mit Ether nach und erhält durch Zugabe von Essigester/1 n Natronlauge die freie Base. Man erhält 60,2 g (65% der Theorie) 2-(4-Chlorphenoxy-methyl)-3,3-dimethyl-1-(1,2,4-triazol-1-yl)-butan-2-ol vom Schmelzpunkt 84 - 87 °C.

*Herstellung des Ausgangsproduktes*

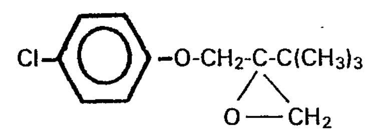

(II-1)

Eine Lösung von 189 ml (2,0 Mol) Dimethylsulfat in 1200 ml absolutem Acetonitril wird bei Raumtemperatur mit einer Lösung von 162 ml (2,2 Mol) Dimethylsulfid in 400 ml absolutem Acetonitril versetzt. Man lässt die Reaktionsmischung über Nacht bei Raumtemperatur rühren. Danach werden 118.8 g (2,2 Mol) Natriummethylat zugegeben. Man lässt 30 Minuten rühren und versetzt dann innerhalb von 30 Minuten tropfenweise mit einer Lösung von 272 g (1,2 Mol) 1-(4-Chlorphenoxy)-3,3-dimethyl-butan-2-on in 600 ml absolutem Acetonitril. Man lässt das Reaktionsgemisch über Nacht nachrühren. Anschliessend wird eingeengt, der Rückstand zwischen Wasser und Essigester verteilt, die organische Phase abgetrennt, zweimal mit Wasser und einmal mit gesättigter Natriumchloridlösung gewaschen, über Natriumsulfat getrocknet, eingeengt und der Rückstand im Vakuum destilliert. Man erhält 242,4 g (84% der Theorie) 2-(4-Chlorphenoxy-methyl)-2-tert.-butyl-oxiran vom Siedepunkt 115-22 °C/-0,003 mm Hg-Säule und vom Schmelzpunkt 50 - 52 °C.

*Beispiel 2*

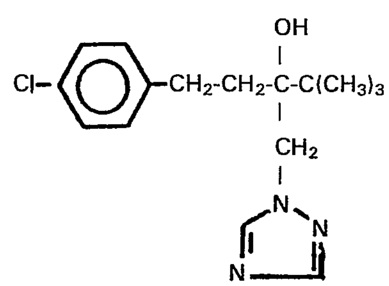

(I-2)

Eine Lösung von 17,9 g (0,075 Mol) 2-(4-Chlorphenyl-ethyl)-2-tert.-butyl-oxiran und 6,9 g (0,1 Mol) 1,2,4-Triazol in 30 ml Ethanol wird 20 Stunden bei 150 °C im Bombenrohr erhitzt. Man lässt abkühlen und engt die Reaktionslösung ein. Der Rückstand

wird in Ether gelöst, dreimal mit Wasser und einmal mit Natriumchloridlösung gewaschen, über Natriumsulfat getrocknet und eingeengt. Der Rückstand wird über eine Kieselgelsäure chromatographiert (Laufmittel: Dichlormethan/Essigester 1:1). Man erhält 12,3 g (53,2% der Theorie) 1-(4-Chlorphenyl)-4,4-dimethyl-3-(1,2,4-triazol-1-yl-methyl)-pentan-3-ol als zähflüssiges Öl.

In analoger Weise wurden die nachfolgenden Verbindungen der allgemeinen Formel I erhalten:

*Tabelle 2*

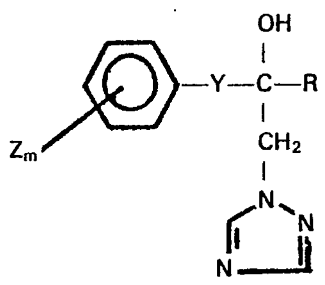

(I)

| Beispiel Nr. | $Z_m$ | Y | R | Schmelzpunkt (°C) |
|---|---|---|---|---|
| I-3 | 4-Cl, 2-$CH_3$ | -O-$CH_2$- | -$C(CH_3)_3$ | 125,5-29 |
| I-4 | 2,4-$Cl_2$ | -O-$CH_2$- | -$C(CH_3)_3$ | 120,5-23,5 |
| I-5 | 4-$CH_3$ | -O-$CH_2$- | -$C(CH_3)_3$ | 98-101,5 |
| I-6 | 2-$CH_3$ | -O-$CH_2$- | -$C(CH_3)_3$ | 89-101 |
| I-7 | 4-F | -$CH_2$-$CH_2$- | -$C(CH_3)_3$ | 91-95,5 |
| I-8 | 4-Cl | -$CH_2$-$CH_2$- | -$C(CH_3)_3$ | 212 (Zers.) (xHCl) |
| I-9 | 4-$CH_3$ | -$CH_2$-$CH_2$- | -$C(CH_3)_3$ | Öl |
| I-10 | 2-Cl | -O-$CH_2$- | -$C(CH_3)_3$ | 109-11 |
| I-11 | 2,4-$Cl_2$ | -$CH_2$-$CH_2$- | -$C(CH_3)_3$ | 94-95 |
| I-12 | 2-$CH_3$ | -$CH_2$-$CH_2$- | -$C(CH_3)_3$ | 82-83 |
| I-13 | 4- 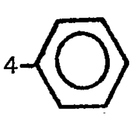 | -O-$CH_2$- | -$C(CH_3)_3$ | 118-19,5 |

*Tabelle 2 (Fortsetzung)*

| Beispiel Nr. | $Z_m$ | Y | R | Schmelzpunkt (°C) |
|---|---|---|---|---|
| I-14 | 4-Cl | -O-$CH_2$- | 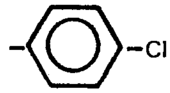 | 81-85 |
| I-15 | 4-Cl | -O-$CH_2$- | 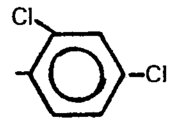 | 149-51 |
| I-16 | 4-F | -O-$CH_2$- | -$C(CH_3)_3$ | 73-75 |
| I-17 | 3-Cl | -O-$CH_2$- | -$C(CH_3)_3$ | 72 |
| I-18 | 2-Cl, 4-F | -O-$CH_2$- | -$C(CH_3)_3$ | 130 |
| I-19 | 3,4-$Cl_2$ | -O-$CH_2$- | -$C(CH_3)_3$ | 124 |
| I-20 | 4- 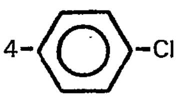 | -O-$CH_2$- | -$C(CH_3)_3$ | 109-11 |
| I-21 | — | -O-$CH_2$- | -$C(CH_3)_3$ | 84-85 |
| I-22 | 4-$OCH_3$ | -O-$CH_2$ | -$C(CH_3)_3$ | 63-66 |
| I-23 | 4-$C(CH_3)_3$ | -O-$CH_2$- | -$C(CH_3)_3$ | 75-78 |
| I-24 | 4-$OCF_3$ | -O-$CH_2$- | -$C(CH_3)_3$ | $n_D^{20}$ = 1,4902 Brechungsindex |

Entsprechend Beispiel 1 werden die nachfolgenden Zwischenprodukte der allgemeinen Formel II erhalten:

*Tabelle 3*

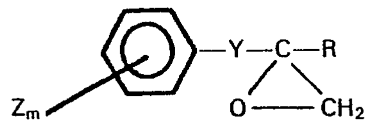 (II)

| Beispiel Nr. | $Z_m$ | Y | R | Siedepunkt (°C)/mm Hg-Säule |
|---|---|---|---|---|
| II-2 | 2,4-$Cl_2$ | $-O-CH_2-$ | $-C(CH_3)_3$ | 125-27/0,3 |
| II-3 | 4-$CH_3$ | $-O-CH_2-$ | $-C(CH_3)_3$ | 85/0,07 |
| II-4 | 2-$CH_3$ | $-O-CH_2-$ | $-C(CH_3)_3$ | 89/0,07 |
| II-5 | 4-Cl, 2-$CH_3$ | $-O-CH_2-$ | $-C(CH_3)_3$ | 114-17/0,33 |
| II-6 | 4-Cl | $-CH_2-CH_2-$ | $-C(CH_3)_3$ | 99-103/0,005 |
| II-7 | 2,4-$Cl_2$ | $-CH_2-CH_2-$ | $-C(CH_3)_3$ | 79/0,004 |
| II-8 | 4-F | $-CH_2-CH_2-$ | $-C(CH_3)_3$ | 78-89/0,003 |
| II-9 | 4-$CH_3$ | $-CH_2-CH_2-$ | $-C(CH_3)_3$ | 74-78/0,003 |
| II-10 | 2-$CH_3$ | $-CH_2-CH_2-$ | $-C(CH_3)_3$ | 95/0,005 |

Die gute Wirksamkeit der erfindungsgemässen Stoffe geht aus den nachfolgenden Beispielen hervor.

In diesen Beispielen werden die nachstehend angegebenen Verbindungen als Vergleichssubstanzen eingesetzt:

(A) = 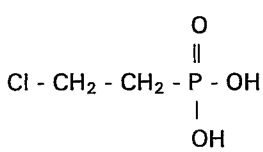

2-Chlorethylphosphonsäure

(B) = $[Cl-CH_2-CH_2-\overset{\oplus}{N}(CH_3)_3]\ \overset{\ominus}{Cl}$

2-Chlorethyl-trimethyl-ammonium-chlorid

(C) = 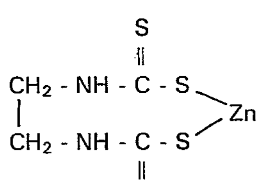

Zink-ethylen-1,2-bisdithiocarbamidat

*Beispiel A*

*Wuchshemmung bei Zuckerrüben*

Lösungsmittel: 30 Gewichtsteile Dimethylformamid
Emulgator: 1 Gewichtsteil Polyoxyethylen-Sorbitan-Monolaurat

Zur Herstellung einer zweckmässigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Zuckerrüben werden im Gewächshaus bis zur vollen Ausbildung der Keimblätter angezogen. In diesem Stadium werden die Pflanzen tropfnass mit den Wirkstoffzubereitungen besprüht. Nach 14 Tagen wird der Zuwachs der Pflanzen gemessen und die Wuchshemmung in Prozent des Zuwachses der Kontrollpflanzen berechnet. Es bedeuten 0% Wuchshemmung ein Wachstum entsprechend dem der Kontrollpflanzen. 100% Wuchshemmung bedeutet Stillstand des Wachstums.

Die erfindungsgemässen Wirkstoffe 1 und 5 zeigen in diesem Test bessere Wuchshemmung als die aus dem Stand der Technik bekannte Substanz (B).

*Beispiel B*

*Wuchshemmung bei Sojabohnen*

Lösungsmittel: 10 Gewichtsteile Methanol
Emulgator: 2 Gewichtsteile Polyoxyethylen-Sorbitan-Monolaurat

Zur Herstellung einer zweckmässigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Junge Sojabohnenpflanzen werden in dem Stadium, in dem die ersten Folgeblätter entfaltet sind, mit den Wirkstoffzubereitungen tropfnass besprüht. Nach 2 Wochen wird der Zuwachs gemessen und die Wuchshemmung in % des Zuwachses der Kontrollpflanzen berechnet. Es bedeuten 100% den Stillstand des Wachstums und 0% ein Wachstum entsprechend dem der unbehandelten Kontrollpflanzen.

Die erfindungsgemässen Wirkstoffe 3 und 1 zeigen in diesem Test eine bessere Wuchshemmung als die aus dem Stand der Technik bekannte Substanz (B).

*Beispiel C*

*Wuchshemmung bei Baumwolle*

Lösungsmittel: 30 Gewichtsteile Dimethylformamid
Emulgator: 1 Gewichtsteil Polyoxyethylen-Sorbitan-Monolaurat

Zur Herstellung einer zweckmässigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächshaus bis zur vollen Entfaltung des 5. Folgeblattes angezogen. In diesem Stadium werden die Pflanzen tropfnass mit den Wirkstoffzubereitungen besprüht. Nach 3 Wochen wird der Zuwachs der Pflanzen gemessen und die Wuchshemmung in Prozent des Zuwachses der Kontrollpflanzen berechnet. Es bedeuten 100% Wuchshemmung den Stillstand des Wachstums und 0% ein Wachstum entsprechend dem der Kontrollpflanzen.

Die erfindungsgemässen Wirkstoffe 1 und 5 zeigen in diesem Test eine bessere Wuchshemmung als die aus dem Stand der Technik bekannte Substanz (A).

*Beispiel D*

*Stimulierung der $CO_2$-Assimilation bei Sojabohnen*

Sojabohnen werden, wie im Beispiel (B) beschrieben, mit den Wirkstoffzubereitungen behandelt. 7 Tage nach der Behandlung wird an Blattscheiben dieser Pflanzen und entsprechender Kontrollpflanzen die $CO_2$-Assimilation mit Hilfe eines Infrarotanalysators gemessen. Die erfindungsgemässen Wirkstoffe 3 und 7 zeigen in den Konzentrationen 250, 500 und 1000 ppm eine gegenüber den Kontrollen deutlich erhöhte $CO_2$-Assimilation. Dieser Effekt lässt Ertragssteigerungen durch den Wirkstoff erwarten.

*Beispiel E*

Erysiphe-Test (Gerste) / protektiv

Lösungsmittel: 100 Gewichtsteile Dimethylformamid
Emulgator: 0,25 Gewichtsteile Alkyl-aryl-polyglykolether

Zur Herstellung einer zweckmässigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp.hordei bestäubt.

Die Pflanzen werden in einem Gewächshaus bei einer Temperatur von ca. 20°C und einer relativen Luftfeuchtigkeit von ca. 80% aufgestellt, um die Entwicklung von Mehltaupusteln zu begünstigen.

7 Tage nach der Inokulation erfolgt die Auswertung.

Eine deutliche Überlegenheit in der Wirksamkeit gegenüben der aus dem Stand der Technik bekannten Substanz (C) zeigen bei diesem Test z.B. die Verbindungen gemäss folgender Herstellungsbeispiele: 3, 4, 1, 5, 6 und 7.

*Beispiel F*

Gerstenmehltau-Test (Erysiphe graminis var.hordei)/systemisch (pilzliche Getreidesprosskrankheit)

Die Anwendung der Wirkstoffe erfolgt als pulverförmige Saatgutbehandlungsmittel. Sie werden hergestellt durch Abstrecken des Wirkstoffes mit einem Gemisch aus gleichen Gewichtsteilen Talkum und Kieselgur zu einer feinpulverigen Mischung mit der gewünschten Wirkstoffkonzentration.

Zur Saatgutbehandlung schüttet man Gerstensaatgut mit dem abgestreckten Wirkstoff in einer verschlossenen Glasflasche. Das Saatgut sät man mit 3 × 12 Korn in Blumentöpfe 2 cm tief in ein Gemisch aus einem Volumenteil Fruhstorfer Einheitserde und einem Volumenteil Quarzsand ein. Die Keimung und der Auflauf erfolgen unter günstigen Bedingungen im Gewächshaus. 7 Tage nach der Aussaat, wenn die Gerstenpflanzen ihr erstes Blatt entfaltet haben, werden sie mit frischen Sporen von Erysiphe graminis var.hordei bestäubt und bei 21-22°C und 80-90% rel. Luftfeuchte und 16-stündiger Belichtung weiter kultiviert. Innerhalb von 6 Tagen bilden sich an den Blättern die typischen Mehltaupusteln aus.

Der Befallsgrad wird in Prozent des Befalls der unbehandelten Kontrollpflanzen ausgedrückt. So bedeutet 0% keinen Befall und 100% den gleichen Befallsgrad wie bei der unbehandelten Kontrolle. Der Wirkstoff ist um so wirksamer je geringer der Mehltaubefall ist.

Eine deutliche Überlegenheit in der Wirksamkeit

gegenüber der aus dem Stand der Technik bekannten Substanz (C) zeigt bei diesem Test z.B. die Verbindung gemäss Herstellungsbeispiel: 3.

**Patentansprüche**

1. 1-Hydroxyethyl-azol-Derivate der allgemeinen Formel I

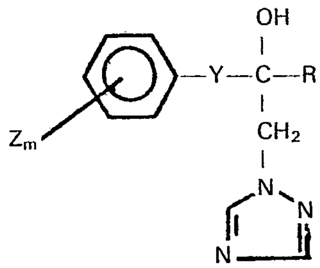

(I)

in welcher

R für Alkyl mit 1 bis 4 Kohlenstoffatomen, gegebenenfalls durch Alkyl mit 1 oder 2 Kohlenstoffatomen substituiertes Cycloalkyl mit 3 bis 7 Kohlenstoffatomen oder für gegebenenfalls durch Halogen, Alkyl mit 1 bis 4 Kohlenstoffatomen und/oder Halogenalkyl mit 1 oder 2 Kohlenstoffatomen und 1 bis 5 Halogenatomen substituiertes Phenyl steht,

Y für die Gruppierungen $-CH_2-CH_2$ und $-O-CH_2$ steht, wobei im Falle der $-OCH_2$-Gruppe das Sauerstoffatom mit dem Phenylrest verbunden ist,

Z für Halogen, Alkyl mit 1 bis 4 Kohlenstoffatomen, Cycloalkyl mit 5 bis 7 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, gegebenenfalls durch Halogen und/oder Alkyl mit 1 bis 4 Kohlenstoffatomen substituiertes Phenyl, gegebenenfalls durch Halogen und/oder Alkyl mit 1 bis 4 Kohlenstoffatomen substituiertes Phenoxy, gegebenenfalls durch Halogen und/oder Alkyl mit 1 bis 4 Kohlenstoffatomen substituiertes Phenylalkyl mit 1 oder 2 Kohlenstoffatomen im Alkylteil oder für gegebenenfalls durch Halogen und/oder Alkyl mit 1 bis 4 Kohlenstoffatomen substituiertes Phenylalkoxy mit 1 oder 2 Kohlenstoffatomen im Alkoxyteil steht und

m für die Zahlen 0, 1, 2 oder 3 steht,

sowie deren Säureadditions-Salze und Metallsalz-Komplexe

2. Verfahren zur Herstellung von 1-Hydroxyethyl-azol-Derivaten der allgemeinen Formel I

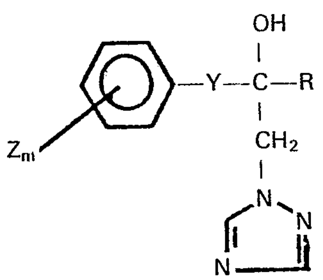

(I)

in welcher

R für Alkyl mit 1 bis 4 Kohlenstoffatomen, gegebenenfalls durch Alkyl mit 1 oder 2 Kohlenstoffatomen substituiertes Cycloalkyl mit 3 bis 7 Kohlenstoffatomen oder für gegebenenfalls durch Halogen, Alkyl mit 1 bis 4 Kohlenstoffatomen und/oder Halogenalkyl mit 1 oder 2 Kohlenstoffatomen und 1 bis 5 Halogenatomen substituiertes Phenyl steht,

Y für die Gruppierungen $-CH_2-CH_2$ und $-O-CH_2$ steht, wobei im Falle der $-OCH_2$-Gruppe das Sauerstoffatom mit dem Phenylrest verbunden ist,

Z für Halogen, Alkyl mit 1 bis 4 Kohlenstoffatomen, Cycloalkyl mit 5 bis 7 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, gegebenenfalls durch Halogen und/oder Alkyl mit 1 bis 4 Kohlenstoffatomen substituiertes Phenyl, gegebenenfalls durch Halogen und/oder Alkyl mit 1 bis 4 Kohlenstoffatomen substituiertes Phenoxy, gegebenenfalls durch Halogen und/oder Alkyl mit 1 bis 4 Kohlenstoffatomen substituiertes Phenylalkyl mit 1 oder 2 Kohlenstoffatomen im Alkylteil oder für gegebenenfalls durch Halogen und/oder Alkyl mit 1 bis 4 Kohlenstoffatomen substituiertes Phenylalkoxy mit 1 oder 2 Kohlenstoffatomen im Alkoxyteil steht und

m für die Zahlen 0, 1, 2 oder 3 steht,

sowie deren Säureadditions-Salzen und Metallsalz-Komplexen, dadurch gekennzeichnet, dass man Oxirane der allgemeinen Formel II

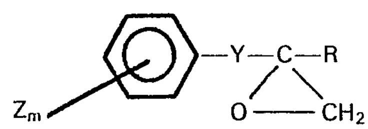

(II)

in welcher

R, Y, Z und m die oben angegebene Bedeutung haben, mit 1,2,4-Triazol der Formel III

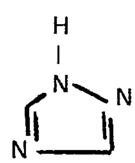

(III)

in Gegenwart eines Verdünnungsmittels und gegebenenfalls in Gegenwart einer Base umsetzt, und gegebenenfalls anschliessend an die erhaltenen Verbindungen der allgemeinen Formel I eine Säure oder ein Metallsalz addiert.

3. Pflanzenwachstumsregulierende und fungizide Mittel, gekennzeichnet durch einen Gehalt an mindestens einem 1-Hydroxyethyl-azol-Derivat der allgemeinen Formel I bzw. einem Säureadditions-Salz oder Metallsalz-Komplex eines 1-Hydroxyethyl-azol-Derivates der allgemeinen Formel I neben Streckmitteln und/oder oberflächenaktiven Stoffen.

4. Verwendung von 1-Hydroxyethyl-azol-Derivaten der allgemeinen Formel I bzw. von Säureadditions-Salzen oder Metallsalz-Komplexen von 1-Hydroxyethyl-azol-Derivaten der allgemeinen Formel I zur Regulierung des Pflanzenwachstums bzw. zur Bekämpfung von Pilzen.

5. Verfahren zur Herstellung von pflanzenwachstumsregulierenden und fungiziden Mitteln, dadurch gekennzeichnet, dass man 1-Hydroxyethyl-azol-Derivate der allgemeinen Formel I bzw. Säureadditions-Salze oder Metallsalz-Komplexe von 1-Hydroxyethyl-azol-Derivaten der allgemeinen Formel I mit Streckmitteln und/oder oberflächenaktiven Stoffen vermischt.

6. Oxirane der allgemeinen Formel IIa

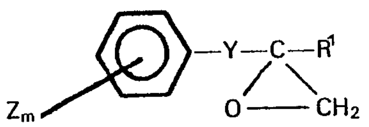

(IIa)

in welcher

$R^1$ für tert.-Butyl, gegebenenfalls durch Alkyl mit 1 oder 2 Kohlenstoffatomen substituiertes Cycloalkyl mit 3 bis 7 Kohlenstoffatomen oder für gegebenenfalls durch Halogen, Alkyl mit 1 bis 4 Kohlenstoffatomen und/oder Halogenalkyl mit 1 oder 2 Kohlenstoffatomen und 1 bis 5 Halogenatomen substituiertes Phenyl steht,

Y für die Gruppierungen $-CH_2-CH_2$ und $-O-CH_2$ steht, wobei im Falle der $-OCH_2$-Gruppe das Sauerstoffatom mit dem Phenylrest verbunden ist,

Z für Halogen, Alkyl mit 1 bis 4 Kohlenstoffatomen, Cycloalkyl mit 5 bis 7 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, gegebenenfalls durch Halogen und/oder Alkyl mit 1 bis 4 Kohlenstoffatomen substituiertes Phenyl, gegebenenfalls durch Halogen und/oder Alkyl mit 1 bis 4 Kohlenstoffatomen substituiertes Phenoxy, gegebenenfalls durch Halogen und/oder Alkyl mit 1 bis 4 Kohlenstoffatomen substituiertes Phenylalkyl mit 1 oder 2 Kohlenstoffatomen im Alkylteil oder für gegebenenfalls durch Halogen und/oder Alkyl mit 1 bis 4 Kohlenstoffatomen substituiertes Phenylalkoxy mit 1 oder 2 Kohlenstoffatomen im Alkoxyteil steht und

m für die Zahlen 0, 1, 2 oder 3 steht.

7. Verfahren zur Herstellung von Oxiranen der allgemeinen Formel IIa

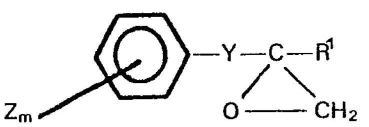

(IIa)

in welcher

$R^1$ für tert.-Butyl, gegebenenfalls durch Alkyl mit 1 oder 2 Kohlenstoffatomen substituiertes Cycloalkyl mit 3 bis 7 Kohlenstoffatomen oder für gegebenenfalls durch Halogen, Alkyl mit 1 bis 4 Kohlenstoffatomen und/oder Halogenalkyl mit 1 oder 2 Kohlenstoffatomen und 1 bis 5 Halogenatomen substituiertes Phenyl steht,

Y für die Gruppierungen $-CH_2-CH_2$ und $-O-CH_2$ steht, wobei im Falle der $-OCH_2$-Gruppe das Sauerstoffatom mit dem Phenylrest verbunden ist,

Z für Halogen, Alkyl mit 1 bis 4 Kohlenstoffatomen, Cycloalkyl mit 5 bis 7 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, gegebenenfalls durch Halogen und/oder Alkyl mit 1 bis 4 Kohlenstoffatomen substituiertes Phenyl, gegebenenfalls durch Halogen und/oder Alkyl mit 1 bis 4 Kohlenstoffatomen substituiertes Phenoxy, gegebenenfalls durch Halogen und/oder Alkyl mit 1 bis 4 Kohlenstoffatomen substituiertes Phenylalkyl mit 1 oder 2 Kohlenstoffatomen im Alkylteil oder für gegebenenfalls durch Halogen und/oder Alkyl mit 1 bis 4 Kohlenstoffatomen substituiertes Phenylalkoxy mit 1 oder 2 Kohlenstoffatomen im Alkoxyteil steht und

m für die Zahlen 0, 1, 2 oder 3 steht,

dadurch gekennzeichnet, dass man Ketone der allgemeinen Formel IV

(IV)

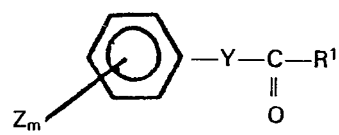

in welcher

$R^1$, Y, Z und m die oben angegebene Bedeutung haben, entweder

α) mit Dimethyloxosulfonium-methylid der Formel V

$$\delta + \delta -$$
$$(CH_3)_2SOCH_2 \qquad (V)$$

in Gegenwart eines Verdünnungsmittels umsetzt, oder

β) mit Trimethylsulfonium-methylsulfat der Formel VI

$$\left[\overset{(+)}{(CH_3)_3S}\right] \quad \overset{(-)}{CH_3SO_4} \qquad (VI)$$

in Gegenwart eines inerten organischen Lösungsmittels sowie in Gegenwart einer Base umsetzt.

**Claims**

1. 1-Hydroxyethyl-azole derivatives of the general formula I

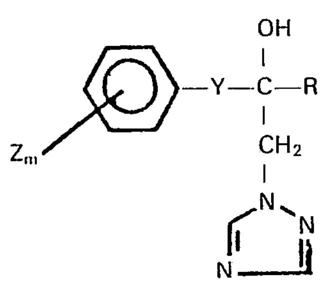

(I)

in which

R represents alkyl with 1 to 4 carbon atoms, cycloalkyl which has 3 to 7 carbon atoms and is optionally substituted by alkyl with 1 or 2 carbon atoms, or phenyl which is optionally substituted by halogen, alkyl with 1 to 4 carbon atoms and/or halogenoalkyl with 1 or 2 carbon atoms and 1 to 5 halogen atoms,

Y represents the grouping $-CH_2-CH_2$ or $-O-CH_2$, the oxygen atom being bonded to the phenyl radical if Y represents the $-OCH_2$ group,

Z represents halogen, alkyl with 1 to 4 carbon atoms, cycloalkyl with 5 to 7 carbon atoms, alkoxy with 1 to 4 carbon atoms, alkylthio with 1 to 4 carbon atoms, halogenoalkyl with 1 or 2 carbon atoms and 1 to 5 halogen atoms, halogenoalkoxy with 1 or 2 carbon atoms and 1 to 5 halogen atoms, halogenoalkylthio with 1 or 2 carbon atoms and 1 to 5 halogen atoms, phenyl which is optionally substituted by halogen and/or alkyl with 1 to 4 carbon atoms, phenoxy which is optionally substituted by halogen and/or alkyl with 1 to 4 carbon atoms, phenylalkyl which has 1 or 2 carbon atoms in the alkyl part and is optionally substituted by halogen and/or alkyl with 1 to 4 carbon atoms, or phenylalkoxy which has 1 or 2 carbon atoms in the alkoxy part and is optionally substituted by halogen and/or alkyl with 1 to 4 carbon atoms, and

m represents the number 0, 1, 2 or 3,

and acid addition salts and metal salt complexes thereof.

2. Process for the preparation of 1-hydroxyethyl-azole derivatives of the general formula I

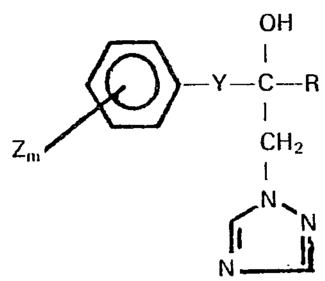

(I)

in which

R represents alkyl with 1 to 4 carbon atoms, cycloalkyl which has 3 to 7 carbon atoms and is optionally substituted by alkyl with 1 or 2 carbon atoms, or phenyl which is optionally substituted by halogen, alkyl with 1 to 4 carbon atoms and/or halogenoalkyl with 1 or 2 carbon atoms and 1 to 5 halogen atoms,

Y represents the grouping $-CH_2-CH_2$ or $-O-CH_2$, the oxygen atom being bonded to the phenyl radical if Y represents the $-OCH_2$ group,

Z represents halogen, alkyl with 1 to 4 carbon atoms, cycloalkyl with 5 to 7 carbon atoms, alkoxy with 1 to 4 carbon atoms, alkylthio with 1 to 4 carbon atoms, halogenoalkyl with 1 or 2 carbon atoms and 1 to 5 halogen atoms, halogenoalkoxy with 1 or 2 carbon atoms and 1 to 5 halogen atoms, halogenoalkylthio with 1 or 2 carbon atoms and 1 to 5 halogen atoms, phenyl which is optionally substituted by halogen and/or alkyl with 1 to 4 carbon atoms, phenoxy which is optionally substituted by halogen and/or alkyl with 1 to 4 carbon atoms, phenylalkyl which has 1 or 2 carbon atoms in the alkyl part and is optionally substituted by halogen and/or alkyl with 1 to 4 carbon atoms, or phenylalkoxy which has 1 or 2 carbon atoms in the alkoxy part and is optionally substituted by halogen and/or alkyl with 1 to 4 carbon atoms, and

m represents the number 0, 1, 2 or 3,

and acid addition salts and metal salt complexes thereof, characterised in that oxiranes of the general formula II

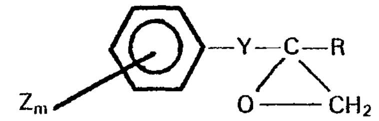

(II)

in which

R, Y, Z and m have the abovementioned meaning, are reacted with 1,2,4-triazole of the formula III

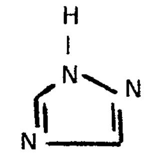

(III)

in the presence of a diluent and if appropriate in the presence of a base, and an acid or a metal salt is then optionally added onto the resulting compounds of the general formula I.

3. Plant growth-regulating and fungicidal agents, characterised in that they contain at least one 1-hydroxyethyl-azole derivative of the general formula I or acid addition salt or metal salt complex of a 1-hydroxyethyl-azole derivative of the general formula I besides extenders and/or surface-active substances.

4. Use of 1-hydroxyethyl-azole derivatives of the general formula I or of acid addition salts or metal salt complexes of 1-hydroxyethyl-azole derivatives of the general formula I, for regulating plant growth or for combating fungi.

5. Process for the preparation of plant growth-regulating and fungicidal agents, characterised in that 1-hydroxyethyl-azole derivatives of the general formula I or acid addition salts or metal salt complexes of 1-hydroxyethyl-azole derivatives of the general formula I are mixed with extenders and/or surface-active substances.

6. Oxiranes of the general formula IIa

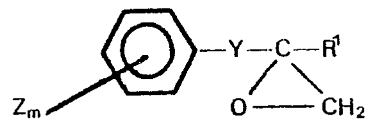

(IIa)

in which

$R^1$ represents tert.-butyl, cycloalkyl which has 3 to 7 carbon atoms and is optionally substituted by alkyl with 1 or 2 carbon atoms, or phenyl which is optionally substituted by halogen, alkyl with 1 to 4 carbon atoms and/or halogenoalkyl with 1 or 2 carbon atoms and 1 to 5 halogen atoms,

Y represents the grouping $-CH_2-CH_2$ or $-O-CH_2$, the oxygen atom being bonded to the phenyl radical if Y represents the $-OCH_2$ group,

Z represents halogen, alkyl with 1 to 4 carbon atoms, cycloalkyl with 5 to 7 carbon atoms, alkoxy

with 1 to 4 carbon atoms, alkylthio with 1 to 4 carbon atoms, halogenoalkyl with 1 or 2 carbon atoms, and 1 to 5 halogen atoms, halogenoalkoxy with 1 or 2 carbon atoms and 1 to 5 halogen atoms, halogenoalkylthio with 1 or 2 carbon atoms and 1 to 5 halogen atoms, phenyl which is optionally substituted by halogen and/or alkyl with 1 to 4 carbon atoms, phenoxy which is optionally substituted by halogen and/or alkyl with 1 to 4 carbon atoms, phenylalkyl which has 1 or 2 carbon atoms in the alkyl part and is optionally substituted by halogen and/or alkyl with 1 to 4 carbon atoms, or phenylalkoxy which has 1 or 2 carbon atoms in the alkoxy part and is optionally substituted by halogen and/or alkyl with 1 to 4 carbon atoms, and

m represents the number 0, 1, 2 or 3.

7. Process for the preparation of oxiranes of the general formula IIa,

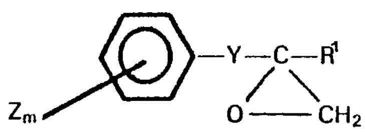

(IIa)

in which

$R^1$ represents tert.-butyl, cycloalkyl which has 3 to 7 carbon atoms and is optionally substituted by alkyl with 1 or 2 carbon atoms, or phenyl which is optionally substituted by halogen, alkyl with 1 to 4 carbon atoms and/or halogenoalkyl with 1 or 2 carbon atoms and 1 to 5 halogen atoms,

Y represents the grouping $-CH_2-CH_2$ or $-O-CH_2$, the oxygen atom being bonded to the phenyl radical if Y represents the $-OCH_2$ group,

Z represents halogen, alkyl with 1 to 4 carbon atoms, cycloalkyl with 5 to 7 carbon atoms, alkoxy with 1 to 4 carbon atoms, alkylthio with 1 to 4 carbon atoms, halogenoalkyl with 1 or 2 carbon atoms, and 1 to 5 halogen atoms, halogenoalkoxy with 1 or 2 carbon atoms and 1 to 5 halogen atoms, halogenoalkylthio with 1 or 2 carbon atoms and 1 to 5 halogen atoms, phenyl which is optionally substituted by halogen and/or alkyl with 1 to 4 carbon atoms, phenoxy which is optionally substituted by halogen and/or alkyl with 1 to 4 carbon atoms, phenylalkyl which has 1 or 2 carbon atoms in the alkyl part and is optionally substituted by halogen and/or alkyl with 1 to 4 carbon atoms, or phenylalkoxy which has 1 or 2 carbon atoms in the alkoxy part and is optionally substituted by halogen and/or alkyl with 1 to 4 carbon atoms, and

m represents the number 0, 1, 2 or 3, characterised in that ketones of the general formula IV

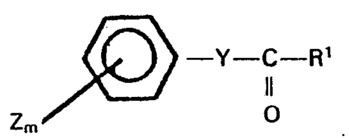

(IV)

in which

$R^1$, Y, Z and m have the abovementioned meaning, are either

α) reacted with dimethyloxosulphonium methylide of the formula V

$$\overset{\delta+\ \ \delta-}{(CH_3)_2SOCH_2} \qquad (V)$$

in the presence of a diluent, or

β) reacted with trimethylsulphonium methyl-sulphate of the formula VI

$$\left[\overset{(+)}{(CH_3)_3S}\right] \quad \overset{(-)}{CH_3SO_4} \qquad (VI)$$

in the presence of an inert organic solvent and in the presence of a base.

## Revendications

1. Dérivés de 1-hydroxyéthyl-azole de formule générale I:

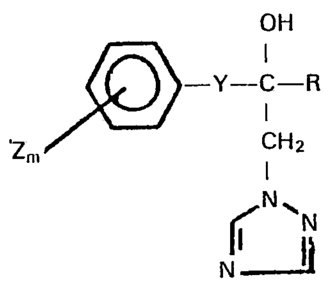

(I)

dans laquelle

R représente un groupe alkyle contenant 1 à 4 atomes de carbone, un groupe cycloalkyle contenant 3 à 7 atomes de carbone et éventuellement substitué par un groupe alkyle contenant 1 ou 2 atomes de carbone, ou un groupe phényle éventuellement substitué par un atome d'halogène, par un groupe alkyle contenant 1 à 4 atomes de carbone et/ou par un groupe halogénalkyle contenant 1 ou 2 atomes de carbone et 1 à 5 atomes d'halogènes,

Y représente les groupements $-CH_2-CH_2-$ et $-O-CH_2-$ et, dans le cas du groupe $-OCH_2$, l'atome d'oxygène est relié au groupe phényle,

Z représente un atome d'halogène, un groupe alkyle contenant 1 à 4 atomes de carbone, un groupe cycloalkyle contenant 5 à 7 atomes de carbone, un groupe alcoxy contenant 1 à 4 atomes de carbone, un groupe alkylthio contenant 1 à 4 atomes de carbone, un groupe halogénalkyle contenant 1 ou 2 atomes de carbone et 1 à 5 atomes d'halogènes, un groupe halogénalcoxy contenant 1 ou 2 atomes de carbone et 1 à 5 atomes d'halogènes, un groupe halogénalkylthio contenant 1 ou 2 atomes de carbone et 1 à 5 atomes d'halogènes, un groupe phényle éventuellement substitué par un atome d'halogène et/ou par un groupe alkyle contenant 1 à 4 atomes de carbone, un groupe phénoxy éventuellement substitué par un atome d'halogène et/ou par un groupe alkyle contenant 1 à 4 atomes de carbone, un groupe phénylalkyle contenant 1 ou 2 atomes de carbone dans la fraction alkyle et éventuellement substitué par un atome d'halogène et/ou par un groupe alkyle contenant 1 à 4 atomes de carbone, ou un groupe phénylalcoxy contenant 1 ou 2 atomes de carbone

dans la fraction alcoxy et éventuellement substitué par un atome d'halogène et/ou par un groupe alkyle contenant 1 à 4 atomes de carbone, et

m représente les nombres 0, 1, 2 ou 3,

ainsi que leurs sels d'addition d'acide et leurs complexes de sels métalliques.

2. Procédé de préparation de dérivés de 1-hydroxyéthyl-azole de formule générale I:

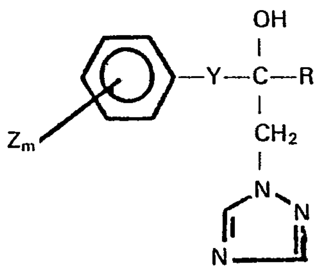

(I)

dans laquelle

R représente un groupe alkyle contenant 1 à 4 atomes de carbone, un groupe cycloalkyle contenant 3 à 7 atomes de carbone et éventuellement substitué par un groupe alkyle contenant 1 ou 2 atomes de carbone, ou un groupe phényle éventuellement substitué par un atome d'halogène, par un groupe alkyle contenant 1 à 4 atomes de carbone et/ou par un groupe halogénalkyle contenant 1 ou 2 atomes de carbone et 1 à 5 atomes d'halogènes,

Y représente les groupements $-CH_2-CH_2-$ et $-O-CH_2-$ et, dans le cas du groupe $-OCH_2$, l'atome d'oxygène est relié au groupe phényle,

Z représente un atome d'halogène, un groupe alkyle contenant 1 à 4 atomes de carbone, un groupe cycloalkyle contenant 5 à 7 atomes de carbone, un groupe alcoxy contenant 1 à 4 atomes de carbone, un groupe alkylthio contenant 1 à 4 atomes de carbone, un groupe halogénalkyle contenant 1 ou 2 atomes de carbone et 1 à 5 atomes d'halogènes, un groupe halogénalcoxy contenant 1 ou 2 atomes de carbone et 1 à 5 atomes d'halogènes, un groupe halogénalkylthio contenant 1 ou 2 atomes de carbone et 1 à 5 atomes d'halogènes, un groupe phényle éventuellement substitué par un atome d'halogène et/ou par un groupe alkyle contenant 1 à 4 atomes de carbone, un groupe phénoxy éventuellement substitué par un atome d'halogène et/ou par un groupe alkyle contenant 1 à 4 atomes de carbone, un groupe phénylalkyle contenant 1 ou 2 atomes de carbone dans la fraction alkyle et éventuellement substitué par un atome d'halogène et/ou par un groupe alkyle contenant 1 à 4 atomes de carbone, ou un groupe phénylalcoxy contenant 1 ou 2 atomes de carbone dans la fraction alcoxy et éventuellement substitué par un atome d'halogène et/ou par un groupe alkyle contenant 1 à 4 atomes de carbone, et

m représente les nombres 0, 1, 2 ou 3,

ainsi que de leurs sels d'addition d'acide et de leurs complexes de sels métalliques, caractérisé en ce qu'on fait réagir des oxirannes de formule générale II:

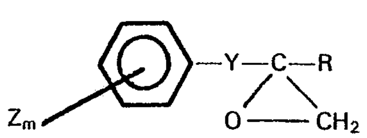

(II)

dans laquelle

R, Y, Z et m ont les significations indiquées ci-dessus, avec le 1,2,4-triazole de formule III:

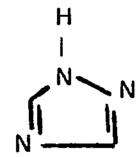

(III)

en présence d'un diluant et éventuellement en présence d'une base puis, aux composés obtenus de formule générale I, on ajoute éventuellement un acide ou un sel métallique.

3. Agents fongicides et de régulation de la croissance des plantes, caractérisé en ce qu'ils contiennent au moins un dérivé de 1-hydroxyéthyl-azole de formule générale I ou un sel d'addition d'acide ou encore un complexe d'un sel métallique d'un dérivé de 1-hydroxyéthyl-azole de formule générale I en plus de diluants et/ou de substances tensio-actives.

4. Utilisation de dérivés de 1-hydroxyéthyl-azole de formule générale I ou de sels d'addition d'acide ou encore de complexes de sels métalliques de dérivés de 1-hydroxyéthyl-azole de formule générale I pour la régulation de la croissance des plantes ou pour combattre les champignons.

5. Procédé de préparation d'agents fongicides et de régulation de la croissance des plantes, caractérisé en ce qu'on mélange des dérivés de 1-hydroxyéthyl-azole de formule générale I ou des sels d'addition d'acide ou encore des complexes de sels métalliques de dérivés de 1-hydroxyéthyl-azole de formule générale I avec des diluants et/ou des substances tensio-actives.

6. Oxirannes de formule générale IIa:

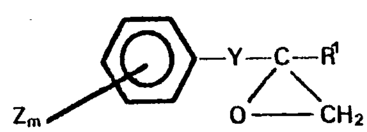

(IIa)

dans laquelle

$R^1$ représente un groupe tert.-butyle, un groupe cycloalkyle contenant 3 à 7 atomes de carbone et éventuellement substitué par un groupe alkyle contenant 1 ou 2 atomes de carbone, ou un groupe phényle éventuellement substitué par un atome d'halogène, par un groupe alkyle contenant 1 à 4 atomes de carbone et/ou par un groupe halogénalkyle contenant 1 ou 2 atomes de carbone et 1 à 5 atomes d'halogènes,

Y représente les groupements $-CH_2-CH_2$ et $-O-CH_2$ et, dans le cas du groupe $-OCH_2$, l'atome d'oxygène est relié au groupe phényle,

Z représente un atome d'halogène, un groupe alkyle contenant 1 à 4 atomes de carbone, un groupe cycloalkyle contenant 5 à 7 atomes de carbone, un groupe alcoxy contenant 1 à 4 atomes de carbone, un groupe alkylthio contenant 1 à 4 atomes de carbone, un groupe halogénalkyle contenant 1 ou 2 atomes de carbone et 1 à 5 atomes d'halogènes, un groupe halogénalcoxy contenant 1 ou 2 atomes de carbone et 1 à 5 atomes d'halogènes, un groupe halogénalkylthio contenant 1 ou 2 atomes de carbone et 1 à 5 atomes d'halogènes, un groupe phényle éventuellement substitué par un atome d'halogène et/ou par un groupe alkyle contenant 1 à 4 atomes de carbone, un groupe phénoxy éventuellement substitué par un atome d'halogène et/ou par un groupe alkyle contenant 1 à 4 atomes de carbone, un groupe phénylalkyle contenant 1 ou 2 atomes de carbone dans la fraction alkyle et éventuellement substitué par un atome d'halogène et/ou par un groupe alkyle contenant 1 à 4 atomes de carbone, ou un groupe phénylalcoxy contenant 1 ou 2 atomes de carbone dans la fraction alcoxy et éventuellement substitué par un atome d'halogène et/ou par un groupe alkyle contenant 1 à 4 atomes de carbone, et

m représente les nombres 0, 1, 2 ou 3.

7. Procédé de préparation d'oxirannes de formule générale IIa:

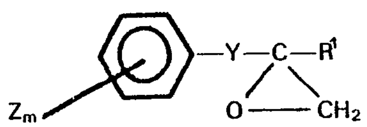

(IIa)

dans laquelle

$R^1$ représente un groupe tert.-butyle, un groupe cycloalkyle contenant 3 à 7 atomes de carbone et éventuellement substitué par un groupe alkyle contenant 1 ou 2 atomes de carbone, ou un groupe phényle éventuellement substitué par un atome d'halogène, par un groupe alkyle contenant 1 à 4 atomes de carbone et/ou par un groupe halogénalkyle contenant 1 ou 2 atomes de carbone et 1 à 5 atomes d'halogènes,

Y représente les groupements $-CH_2-CH_2$ et $-O-CH_2$ et, dans le cas du groupe $-OCH_2$, l'atome d'oxygène est relié au groupe phényle,

Z représente un atome d'halogène, un groupe alkyle contenant 1 à 4 atomes de carbone, un groupe cycloalkyle contenant 5 à 7 atomes de carbone, un groupe alcoxy contenant 1 à 4 atomes de carbone, un groupe alkylthio contenant 1 à 4 atomes de carbone, un groupe halogénalkyle contenant 1 ou 2 atomes de carbone et 1 à 5 atomes d'halogènes, un groupe halogénalcoxy contenant 1 ou 2 atomes de carbone et 1 à 5 atomes d'halogènes, un groupe halogénalkylthio contenant 1 ou 2 atomes de carbone et 1 à 5 atomes d'halogènes, un groupe phényle éventuellement substitué par un atome d'halogène et/ou par un groupe alkyle contenant 1 à 4 atomes de carbone, un groupe phénoxy éventuellement substitué par un atome d'halogène et/ou par un groupe alkyle contenant 1 à 4 atomes de carbone, un groupe phénylalkyle contenant 1 ou 2 atomes de carbone dans la fraction alkyle et éventuellement substitué par un atome d'halogène et/ou par un groupe alkyle contenant 1 à 4 atomes de carbone, ou un groupe phénylalcoxy contenant 1 ou 2 atomes de carbone dans la fraction alcoxy et éventuellement substitué par un atome d'halogène et/ou par un groupe alkyle contenant 1 à 4 atomes de carbone, et

m représente les nombres 0, 1, 2 ou 3, caractérisé en ce qu'on fait réagir des cétones de formule générale IV:

(IV)

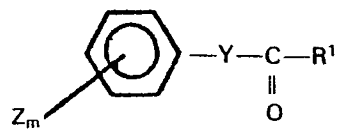

dans laquelle

$R^1$, Y, Z et m ont les significations indiquées cidessus:

α) avec du méthylure de diméthyloxosulfonium de formule V:

$$\overset{\delta+}{(CH_3)_2SO}\overset{\delta-}{CH_2} \qquad (V)$$

en présence d'un diluant, ou

β) avec du méthyl-sulfate de triméthyl-sulfonium de formule VI:

$$\left[(CH_3)_3\overset{(+)}{S}\right] \quad \overset{(-)}{CH_3SO_4} \qquad (VI)$$

en présence d'un solvant organique inerte, ainsi qu'en présence d'une base.